# FASCICULE DE BREVET EUROPEEN

(11) **EP 1 382 361 B1**
(45) Date de publication et mention de la délivrance du brevet: **08.08.2007**
(21) Numéro de dépôt: 03291430.1
(22) Date de dépôt: 13.06.2003
(51) Int. Cl.: A61M 1/36, B01D 71/54, A61M 1/02, B01D 39/16, B01D 67/00, B29C 59/14

(54) **Unité de déleucocytation sélective d'un fluide contenant des plaquettes sanguines**
Einheit zur selektiven Entfernung von Leukozyten aus einem Plättchenprodukt
Unit for the selective removal of leucocytes from a platelet product

(30) Priorité: 10.07.2002 FR 0208687
(43) Date de publication de la demande: 21.01.2004
(73) Titulaire: MACO PHARMA, 59420 Mouvaux (FR)
(72) Inventeur: Chollet, Stéphane, 59420 Mouvaux (FR); Verpoort, Thierry, 59420 Mouvaux (FR)
(74) Mandataire: Geismar, Thierry

(56) Documents cités:
- EP-A- 0 526 678
- WO-A-93/04763
- WO-A-94/17904
- FR-A- 2 821 762
- US-A- 4 927 676
- DATABASE WPI Week 200036 Derwent Publications Ltd., London, GB; AN 2000-415440 XP002236105 & JP 2000 135422 A (ASAHI MEDICAL CO LTD), 16 mai 2000 (2000-05-16)

## Description

L'invention concerne une unité de filtration destinée à permettre la déleucocytation sélective d'un fluide contenant des plaquettes sanguines, ainsi que des systèmes à poches comprenant une telle unité.

Elle s'applique typiquement à la filtration du sang ou d'un composant sanguin contenant des plaquettes tel qu'un plasma riche en plaquettes (PRP) ou un concentré plaquettaire (CP) ainsi qu'à la séparation et au recueil des composants sanguins, notamment en circuit clos.

Le sang total est un tissu constitué de deux types de composants : les cellules sanguines comprenant les globules rouges, les leucocytes et les plaquettes, et le plasma, liquide jaune pâle dans lequel les cellules sanguines sont en suspension.

Actuellement, on ne transfuse aux patients que les composants sanguins nécessaires à leur état. Par exemple, on ne transfuse que les concentrés plaquettaires aux patients atteints de thrombocytopénie, c'est-à-dire dont la teneur en plaquettes dans le sang est réduite.

Les composants sanguins sont habituellement séparés par deux types de centrifugation. La centrifugation dite douce du sang total conduit à le séparer en deux couches : une couche inférieure riche en globules rouges appelée concentré de globules rouges (CGR) ; et une couche supérieure contenant le plasma, les plaquettes et les leucocytes appelée plasma riche en plaquettes (PRP). La centrifugation dite dure conduit à une séparation en trois couches : une couche inférieure de CGR ; une couche supérieure de plasma pauvre en plaquettes (PPP) ; et une couche intermédiaire formée essentiellement de leucocytes et de plaquettes, dénommée couche leuco-plaquettaire ou buffy coat. Le concentré plaquettaire (CP) est obtenu par centrifugation du PRP ou de la couche leuco-plaquettaire.

Il s'est avéré que les leucocytes ont des effets indésirables très importants, ce qui a conduit à chercher à les éliminer des composants sanguins destinés à la transfusion. En effet, les leucocytes augmentent les risques de rejet immunitaire tel que le GVHD (Graft Versus Host Disease : rejet de la greffe par l'hôte) et favorisent la transmission d'agents infectieux. Il a aussi été montré que les leucocytes affectaient de façon négative la conservation des plaquettes.

On connaît déjà des unités de filtration comprenant un milieu de déleucocytation du sang total et/ou de chacun des composants sanguins. Cependant, la plupart de ces milieux retiennent les leucocytes mais également les plaquettes de sorte qu'ils ne permettent pas d'obtenir un filtrat formé d'un produit plaquettaire exempt de leucocyte.

Pour permettre l'élimination sélective des leucocytes et le passage des plaquettes, on a proposé plusieurs traitements chimiques et/ou physiques de milieux filtrants connus.

Par exemple, il a été envisagé de traiter le milieu filtrant par revêtement ou greffage par radiation de différentes substances chimiques de sorte à limiter l'adhésion des plaquettes sur le milieu filtrant. Ces traitements ont l'inconvénient d'utiliser des réactifs organiques qui peuvent se retrouver dans le sang ou le composant sanguin déleucocyté.

Par ailleurs, il a été envisagé, par exemple dans le document WO-93/04763, de traiter par plasma gazeux le milieu de filtration de sorte à diminuer la rétention des plaquettes. Toutefois, pour être efficace, ce type de traitement nécessite des conditions opératoires, notamment en terme de temps, qui sont particulièrement contraignantes.

Le document WO-94/17904 décrit des membranes en polyuréthanne pour dialyse, modifiées par plasma basse-pression. Cette modification permet de réduire l'activation du complément, et notamment le complément C5a, et la thrombogénicité du sang lors de la dialyse. Ce document ne concerne pas la filtration du sang.

Pour résoudre ces inconvénients, la demanderesse a effectué des essais intensifs et a sélectionné un milieu de déleucocytation formé d'un matériau à biocompatibilité améliorée, c'est-à-dire possédant des caractéristiques intrinsèques de non-rétention des plaquettes sanguines, ledit matériau étant sous forme d'au moins une couche de non-tissé de sorte à améliorer sa capacité d'élimination des leucocytes et ledit milieu ayant été traité par plasma gazeux de sorte à améliorer son hydrophilie.

En particulier, l'unité de filtration incorporant un tel milieu de déleucocytation permet d'obtenir une perte en plaquettes inférieure à 20% et un taux de déleucocytation supérieur à 2 Log.

En outre, l'unité de filtration peut être incorporée dans des systèmes à poches permettant la filtration des fluides contenant des plaquettes, et éventuellement la séparation en circuit clos des différents composants sanguins.

A cet effet, et selon un premier aspect, l'invention propose une unité de filtration destinée à permettre la déleucocytation sélective d'un fluide contenant des plaquettes sanguines tel que le sang ou un composant sanguin, du type comprenant une enveloppe extérieure munie d'au moins un orifice d'entrée et d'au moins un orifice de sortie, l'enveloppe renfermant un élément poreux comprenant un milieu de déleucocytation par adsorption et/ou par filtration des leucocytes, ledit milieu étant formé d'au moins une couche de non-tissé à base de polyuréthanne qui a été traitée par plasma gazeux.

Selon une réalisation, le milieu de déleucocytation comprend en outre au moins une membrane placée en aval dudit milieu.

Selon un second aspect, l'invention concerne un système à poches pour la déleucocytation sélective d'un fluide contenant des plaquettes sanguines, ledit système comprenant :
- une poche de collecte destinée à recevoir le fluide à filtrer, ladite poche étant reliée, par l'intermédiaire d'une tubulure et au niveau d'un orifice de sortie, à un orifice d'entrée d'une unité de filtration selon l'invention ; et
- une poche de recueil du filtrat, ladite poche étant reliée, par l'intermédiaire d'une tubulure et au niveau d'un orifice d'entrée, à un orifice de sortie de l'unité de filtration.

Selon une réalisation particulière, le système à poches est destiné à recueillir et à filtrer un fluide contenant des plaquettes, notamment un ensemble de couches leuco-plaquettaires ou de concentrés plaquettaires.

Selon une autre réalisation particulière, le système à poches est destiné à la séparation des composants sanguins du sang total et à la filtration d'un fluide contenant des plaquettes, notamment le PRP, en circuit clos.

D'autres objets et avantages de l'invention apparaîtront au cours de la description qui suit faite en référence aux dessins annexés.
La figure 1 représente en vue de face une unité de filtration selon un mode de réalisation de l'invention.
La figure 2 représente de façon schématique et en coupe longitudinale l'unité de filtration de la figure 1.
La figure 3 représente en vue schématique un système à poches pour la déleucocytation sélective d'un fluide contenant des plaquettes selon un mode de réalisation.
La figure 4 représente en vue schématique un système à poches pour la séparation des composants sanguins et la déleucocytation sélective d'un fluide contenant des plaquettes selon un mode de réalisation.

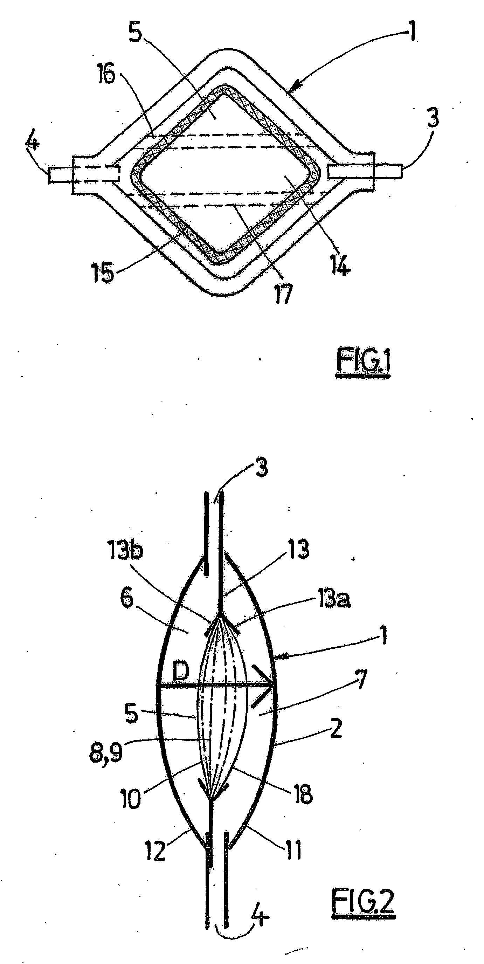

Sur les figures 1 et 2 est représentée une unité de filtration 1 destinée à permettre la déleucocytation sélective d'un fluide contenant des plaquettes et la récupération des plaquettes dans le fluide filtré.

Un fluide contenant des plaquettes peut être par exemple un sang total, un plasma riche en plaquettes (PRP), une couche leuco-plaquettaire ou un concentré plaquettaire.

Notamment, l'unité de filtration 1 est destinée à filtrer une unité de PRP, c'est-à-dire la quantité de PRP obtenue après centrifugation d'un don de sang total. L'unité de filtration peut aussi servir à filtrer un ensemble de couche leuco-plaquettaire ou de concentrés plaquettaires (CP), c'est-à-dire la quantité de couches leuco-plaquettaire ou de CP obtenue après la réunion de deux à huit unités de couche leuco-plaquettaire ou de CP issus d'un don de sang total.

Dans le mode de réalisation représenté sur les figures 1 et 2, l'unité de filtration 1 comprend une enveloppe extérieure 2 munie d'un orifice d'entrée 3 destiné à recevoir le fluide à filtrer et d'un orifice de sortie 4 destiné à recueillir le filtrat, entre lesquels le fluide s'écoule suivant une direction D.

L'unité de filtration 1 comprend en outre un élément poreux 5 disposé dans l'enveloppe extérieure 2 de sorte à délimiter un compartiment d'entrée 6 en communication avec l'orifice d'entrée 3 et un compartiment de sortie 7 en communication avec l'orifice de sortie 4.

Dans la description, les termes « amont » et « aval » sont définis par rapport au sens de circulation du fluide dans l'unité de filtration 1.

L'élément poreux 5 comprend un milieu de déleucocytation 8 capable d'éliminer par adsorption et/ou filtration les leucocytes présents dans un produit plaquettaire. Le milieu de déleucocytation 8 est capable de laisser passer les plaquettes, c'est-à-dire que les plaquettes n'adhèrent pas à la surface du milieu de déleucocytation 8.

Le milieu de déleucocytation 8 est notamment multicouche, c'est-à-dire qu'il comprend plusieurs couches 9. Selon une réalisation, les couches 9 ont un diamètre moyen de pores qui décroît selon la direction de l'écoulement D de sorte à créer un gradient de porosité.

Ce gradient de porosité permet d'améliorer la capacité de rétention des leucocytes présents dans le fluide contenant des plaquettes à filtrer en évitant le blocage ou le colmatage du milieu de déleucocytation 8.

Le gradient de porosité peut varier entre 3 et 15 µm, de façon continue ou de façon discrète.

La surface de filtration de l'élément poreux 5 dépend de la quantité de fluide contenant des plaquettes à filtrer et du type de filtration utilisée, c'est-à-dire par gravité ou sous pression. Lorsque le fluide à filtrer est un PRP, la surface de filtration du filtre est par exemple comprise entre 15 et 58 cm², notamment 55 cm². Lorsque le fluide à filtrer est constitué de une à quatre unités de concentrés plaquettaires, la surface de filtration peut être comprise entre 15 et 35 cm², notamment 20 cm².

Le milieu de déleucocytation 8 comprend au moins une couche formée d'un matériau non-tissé à base de polyuréthanne.

Les couches de non-tissé de polyuréthanne contenues dans l'unité de filtration 1 présentent l'avantage de posséder une biocompatibilité suffisante pour que les plaquettes n'adhèrent pas à celui-ci, et ce en présentant une capacité améliorée de rétention des leucocytes.

Les couches de non-tissé de polyuréthanne sont traitées par plasma gazeux avant introduction dans l'unité de filtration 1. Ce traitement permet d'augmenter l'hydrophilie du milieu et donc l'amorçage de la filtration par simple gravité sans avoir recours à une filtration sous pression. Cependant, la filtration sous pression reste envisageable et permet d'augmenter la vitesse de filtration tout en limitant la perte en volume. Suivant l'invention, l'utilisation d'un revêtement polymérique ou copolymérique ou un greffage par radiation d'un polymère ou copolymère pour éviter que les plaquettes n'adhèrent aux couches de non-tissé de polyuréthanne, n'est donc pas nécessaire.

Le diamètre moyen des pores d'un non-tissé de polyuréthanne varie entre 5 et 15 µm, notamment 7 et 15 µm. Le non-tissé de polyuréthanne peut éventuellement subir une compression de type calandrage pour contrôler la taille de ses pores.

Selon une autre réalisation, le milieu de déleucocytation 8 comprend en outre au moins une membrane capable de retenir les leucocytes et de laisser passer les plaquettes. Par exemple, la membrane peut être réalisée à partir d'un matériau choisi parmi les polymères de polymères fluorocarbonés tel que le PVDF, les celluloses, les polyuréthannes, les polysulfones, les polyéthers sulfonés, le polycarbonate et notamment le copolymère polyvinylpyrrolidone/polysulfone (PVP/PSU).

Le copolymère PVP/PSU est notamment constitué de 5% de PVP et de 95% de PSU.

Le diamètre des pores de la membrane varie de 2 à 10 µm. La membrane est placée en aval des couches de polyuréthanne non-tissé.

La nature et les propriétés physico-chimiques de la membrane utilisée peuvent être telles que la membrane ne nécessite aucun traitement chimique et/ou physique afin d'augmenter son hydrophilie. La membrane assure une filtration dite écran, c'est-à-dire que ses pores sont calibrés pour laisser passer les plaquettes tout en retenant les leucocytes.

Dans la réalisation particulière représentée sur les figures 1 et 2, l'enveloppe extérieure 2 est souple et formée par l'assemblage de deux feuilles 11, 12 de matière plastique souple assemblées mutuellement, par exemple, par soudage sur leur périphérie.

L'élément poreux 5 est maintenu dans l'enveloppe extérieure 2 par des moyens d'association étanches déformables qui sont formés d'un cadre souple 13.

Le cadre souple 13 est formé par un assemblage de deux feuilles 13a et 13b, par exemple plastifiées, entre lesquelles est placé l'élément poreux 5.

Ces deux feuilles 13a, 13b sont ajourées dans leur partie centrale et comportent chacune au moins une ouverture 14 permettant le passage du fluide à filtrer.

Les deux feuilles 13a, 13b sont fixées entre elles de préférence au niveau de la périphérie de l'élément poreux 5, par exemple par un cordon de soudure 15, réalisé au travers l'élément poreux 5, assurant à la fois la fixation de l'élément poreux 5 mais également l'étanchéité.

La soudure des feuilles 13a, 13b à travers l'élément poreux 5 provoque une compression, formant un cordon étanche autour de l'élément poreux 5.

Le cadre souple 13 est soudé sur sa périphérie avec les feuilles 11 et 12 formant l'enveloppe extérieure 2, mutuellement sur tout leur pourtour et au niveau de leur périphérie, assurant ainsi l'étanchéité.

Lors de ce soudage, l'orifice d'entrée 3, formé d'une portion de tubulure, est disposé d'un côté du cadre souple 13 et l'orifice de sortie 4, formé d'une autre portion de tubulure, est disposé de l'autre côté du cadre souple 13.

Pour éviter que l'élément poreux ne se colle contre l'enveloppe extérieure 2 et gène ainsi l'écoulement du fluide, deux joncs d'écartement 16,17 sont placés à l'intérieur du compartiment de sortie 7, entre l'élément poreux 5 et l'enveloppe extérieure 2.

Les joncs peuvent être réalisés à partir de tubulures souples soudées par exemple au niveau de la paroi intérieure de la feuille de l'enveloppe extérieure 2, par exemple au niveau de la soudure périphérique.

Il va de soi que le nombre de joncs d'écartement 16,17 peut varier en fonction par exemple des dimensions de l'unité de filtration 1.

Dans un autre mode de réalisation (non représenté), l'enveloppe extérieure est rigide, par exemple réalisée en matériau plastique rigide tel que le polycarbonate.

Comme il est décrit ci dessus en relation avec les figures 1 et 2, l'élément poreux 5 est maintenu dans un cadre souple 13 au moyen d'un cordon de soudure 15. Afin de permettre la soudure de l'élément poreux avec les feuilles 13a,13b formant le cadre souple 13, les matériaux de l'élément poreux en contact avec le cadre souple 13 doivent être soudables.

L'élément poreux 5 peut comprendre un pré-filtre 10 en amont du milieu de déleucocytation 8 afin d'éliminer les particules de type gel ou micro-agrégats, les gros leucocytes et de prévenir le colmatage du milieu de déleucocytation 8. En outre, il peut également comprendre un post-filtre 18 prévu en aval du milieu de déleucocytation 8.

Le pré-filtre 10 et/ou le post-filtre 18 est formé d'un matériau choisi parmi le polyester, le polypropylène, le polyéthylène ou le polyuréthanne, sous forme d'un tissé, d'un tricot ou d'un non-tissé, avec une taille de pores suffisante pour éviter la rétention des plaquettes.

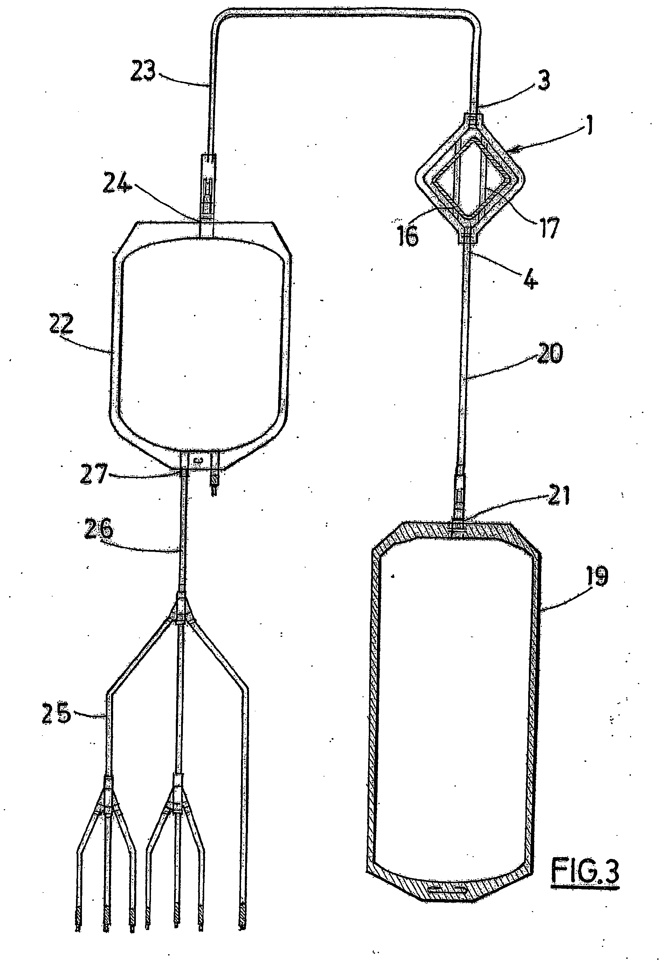

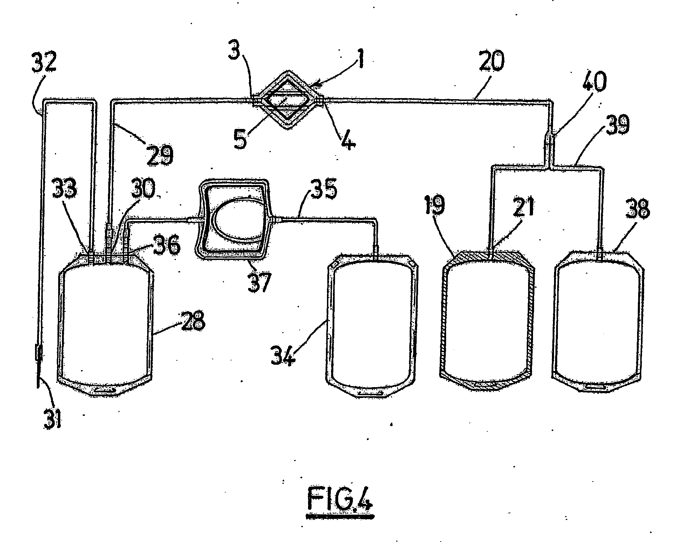

On décrit maintenant en relation avec les figures 3 et 4, deux modes de réalisation d'un système à poches pour la déleucocytation d'un fluide contenant des plaquettes qui comprend une poche 19 destinée à recueillir le filtrat, reliée par l'intermédiaire d'une première tubulure 20 et au niveau de l'orifice d'entrée 21 de ladite poche 19, à un orifice de sortie 4 de l'unité de filtration.

La poche 19 destinée à recueillir le filtrat est souple et comprend un matériau biocompatible ayant une perméabilité suffisante pour maintenir les plaquettes viables pendant leur conservation. Ladite poche 19 est réalisée notamment en PVC plastifié par du trimellitate de tri 2-éthylhéxyle ou en éthylène-acétate de vinyle (EVA).

Dans la description ci-dessus, les tubulures sont notamment souples, soudables et sécables et les liaisons entre les différents éléments des systèmes à poches sont fixes et étanches.

En relation avec la figure 3 et pour la filtration au travers de la même unité de filtration 1 d'un ensemble de couche leuco-plaquettaire, respectivement de concentrés plaquettaires, une poche 22 destinée à contenir le fluide à filtrer est reliée par l'intermédiaire d'une deuxième tubulure 23 et au niveau de l'orifice de sortie 24 de ladite poche 22, à un orifice d'entrée 3 de l'unité de filtration 1.

Le système comprend en outre des moyens de connexion 25 connectés, par l'intermédiaire d'une troisième tubulure 26, à un orifice d'entrée 27 de la poche 22 destinée à contenir le fluide à filtrer.

Les moyens de connexion 25 sont destinés à connecter une ou plusieurs poches (non représentées) contenant des unités de couche leuco-plaquettaire, respectivement de concentrés plaquettaires, et éventuellement une ou plusieurs poches contenant une solution de conservation des plaquettes.

De telles solutions de conservation des plaquettes sont disponibles sur le marché. Une solution de conservation des plaquettes particulière comprend 2,94 g de citrate de sodium 2H₂O, 4,08 g d'acétate de sodium 3H₂O, 6,75 g de chlorure de sodium dans 100 mL d'eau ppi, à un pH 7,2.

Les moyens de connexions sont par exemple des perforateurs ou des joncs pour connexion stérile.

Dans un mode particulier de mise en oeuvre du système représenté sur la figure 3, sept poches contenant chacune une unité de couche leuco-plaquettaire sont mises en communication fluidique par l'intermédiaire des moyens de connexions 25, avec la poche 22 destinée à contenir le fluide à filtrer. Le système à poches est ensuite placé dans une centrifugeuse de façon à ce que l'orifice de sortie 24 de ladite poche 22 soit dirigé vers le haut. Ladite poche 22 est ensuite centrifugée de façon à obtenir d'une part les plaquettes en surnageant et d'autre part les leucocytes et les globules rouges dans le culot. Le surnageant est enfin envoyé par l'orifice de sortie 24 de ladite poche 22 vers la poche 19 destinée à recevoir le filtrat par l'intermédiaire de l'unité de filtration 1. On récupère ainsi des concentrés plaquettaires déleucocytés dans la poche 19 de recueil du filtrat.

Dans un autre mode particulier de mise en oeuvre du système représenté sur la figure 3, six poches contenant chacune une unité de concentré plaquettaire et une septième poche contenant une solution de conservation des plaquettes sont mises en communication fluidique par l'intermédiaire des moyens de connexion 25, avec la poche 22 destinée à contenir le fluide à filtrer. Le mélange concentrés plaquettaires et solution de conservation des plaquettes est ensuite envoyé par l'orifice de sortie 24 de ladite poche 22 vers la poche 19 destinée à recevoir le filtrat par l'intermédiaire de l'unité de filtration 1. On récupère ainsi des concentrés plaquettaires déleucocytés dans la poche 19 de recueil du filtrat.

Dans un autre mode de réalisation (non représenté), les moyens de connexions 25 sont directement reliés par l'intermédiaire de la deuxième tubulure 23 à l'orifice d'entrée 3 de l'unité de filtration 1. Dans ce cas, on connecte directement des concentrés plaquettaires obtenus par centrifugation de couche leuco-plaquettaire.

On décrit maintenant en relation avec la figure 4 un autre mode de réalisation d'un système à poches selon l'invention pour la séparation des composants sanguins et la déleucocytation d'un PRP en circuit clos.

Outre l'unité de filtration et la poche 19 destinée à recueillir le filtrat, le système à poches comprend une poche de collecte 28 contenant un anticoagulant de type ACD ou CPD, reliée par l'intermédiaire d'une quatrième tubulure 29, à l'orifice de sortie 30 de l'unité de filtration 1 et un dispositif de prélèvement du sang 31 relié par l'intermédiaire d'une cinquième tubulure 32 à un orifice d'entrée 33 de la poche de collecte 28.

De plus, le système comprend au moins une poche satellite 34 contenant une solution de conservation des globules rouges de type SAGM reliée au moyen d'une sixième tubulure 35 à un second orifice de sortie 36 de la poche de collecte 22.

La tubulure 35 peut comprendre une unité de filtration 37 destinée à éliminer les leucocytes des globules rouges.

Eventuellement, une poche de recueil du plasma 38 est en communication fluidique avec la poche de recueil du filtrat 19, au moyen d'une septième tubulure 39 par l'intermédiaire d'un connecteur 40 à la première tubulure 20.

Dans un mode particulier non représenté, le système comprend également une poche contenant une solution de conservation des plaquettes en communication fluidique avec la poche de recueil du filtrat 19.

Selon un mode particulier de mise en oeuvre du système selon la figure 4, le sang total est prélevé au moyen du dispositif de prélèvement 31 dans la poche de collecte 28. Le système dans son ensemble est ensuite centrifugé de façon à obtenir dans la poche de collecte 28 une couche inférieure de concentré de globules rouges et une couche supérieure de PRP. Le PRP est envoyé dans la poche 19 de recueil du filtrat par l'intermédiaire de l'unité de filtration 1. Le concentré de globules rouges reste dans la poche de collecte 28 est envoyé dans la poche satellite 34 par l'intermédiaire du filtre 37 à déleucocyter les globules rouges. La tubulure 20 est ensuite scellée et coupée afin de séparer les poches 19 de recueil du filtrat et 38 de recueil du plasma du reste du système. Après centrifugation, on obtient une couche supérieure de plasma qui est envoyée dans la poche de recueil du plasma 38, et une couche inférieure de concentré plaquettaire.

On donne ci-dessous deux exemples de réalisation d'une unité de filtration sélective suivant l'invention.

### Exemple 1

Une unité de filtration 1 telle que représentée sur les figures 1 et 2 comprenant un élément poreux 5 constitué de :
- une couche de non-tissé de polyester ayant une porosité moyenne de 35 µm,
- quatre couches de non-tissé de polyuréthanne de taille moyenne de pores égale à 13,6 µm,
- cinq couches de membrane de PVP/PSU de porosité moyenne comprise entre 4,5 µm et 5,5 µm, et
- une couche de non-tissé de polyester ayant une porosité moyenne de 35 µm.

Le non-tissé de polyuréthanne est traité au plasma gazeux couche par couche dans les conditions opératoires suivantes :
- Puissance : 1000 Watts ;
- Radiofréquence : 13,56 MHz ;
- Temps de traitement : 30 secondes ;
- Pression : 200 à 220 millitores ;
- Débit de O₂ : 1 à 2 litres par minute.

La surface de filtration est de 20 cm².

On forme un ensemble de concentrés plaquettaires dans la poche 22 d'un système à poches tel que représenté sur la figure 3, puis on fait passer l'ensemble dans l'unité de filtration 1 décrite ci-dessus.

La perte en plaquettes mesurée est inférieure à 15% et le taux de déplétion en leucocytes est supérieur à 2 log.

### Exemple 2

Une unité de filtration 1 telle que représentée sur les figures 1 et 2 comprenant un élément poreux 5 constitué de :
- une couche de non-tissé de polyester ayant une porosité moyenne de 35 µm,
- dix couches de non-tissé de polyuréthanne de taille moyenne de pores égale à 8 µm,
- une couche de non-tissé de polyester ayant une porosité moyenne de 35 µm.

Le traitement au plasma est réalisé dans les mêmes conditions opératoires que dans l'exemple 1 et les résultats obtenus sont analogues.

## Revendications

1. Unité de filtration (1) destinée à permettre la déleucocytation sélective d'un fluide contenant des plaquettes sanguines tel que le sang ou un composant sanguin, du type comprenant une enveloppe extérieure (2) munie d'au moins un orifice d'entrée (3) et d'au moins un orifice de sortie (4), l'enveloppe (2) renfermant un élément poreux (5) comprenant un milieu de déleucocytation (8) par adsorption et/ou par filtration des leucocytes, **caractérisée en ce que** ledit milieu est formé d'au moins une couche (9) de non-tissé à base de polyuréthanne qui a été traitée par plasma gazeux.

2. Unité de filtration selon la revendication 1, **caractérisée en ce que** le diamètre moyen des pores de la ou des couche(s) (9) de non-tissé est compris entre 5 et 15 µm.

3. Unité de filtration selon la revendication 1 ou 2, **caractérisé en ce que** le milieu de déleucocytation (8) comprend plusieurs couches (9) empilées qui présentent une porosité décroissante de façon continue ou de façon discrète dans le sens d'écoulement (D) du fluide.

4. Unité de filtration selon l'une quelconque des revendications 1 à 3, **caractérisée en ce que** le milieu de déleucocytation (8) comprend en outre au moins une membrane placée en aval dudit milieu.

5. Unité de filtration selon la revendication 4, **caractérisée en ce que** la ou les membrane(s) est(sont) formées à base d'un matériau choisi dans le groupe comprenant les polymères de polymères fluorocarbonés, les dérivés de cellulose, les polyuréthannes, les polysulfones, les polyéthers sulfonés, le polycarbonate.

6. Unité de filtration selon la revendication 5, **caractérisée en ce que** la ou les membrane(s) est(sont) formée(s) à base d'un copolymère de polyvinylpyrrolidone/polysulfone.

7. Unité de filtration selon l'une quelconque des revendications 4 à 6, **caractérisée en ce que** la ou les membrane(s) présente(nt) un diamètre de pores compris entre 2 et 10 µm.

8. Unité de filtration selon l'une quelconque des revendications 1 à 7, **caractérisée en ce que** l'élément poreux (5) comprend en outre un pré-filtre (10) et/ou un post-filtre (18) qui sont réalisés sous la forme d'au moins une couche de non-tissé et qui sont disposés respectivement coté aval et coté amont du milieu de déleucocytation (8).

9. Système à poches pour la déleucocytation sélective d'un fluide contenant des plaquettes sanguines, **caractérisé en ce qu'**il comprend :
- une poche de collecte (28, 22) destinée à recevoir le fluide à filtrer, ladite poche étant reliée, par l'intermédiaire d'une tubulure et au niveau d'un orifice de sortie, à un orifice d'entrée (3) d'une unité de filtration (1) selon l'une quelconque des revendications 1 à 8 ; et
- une poche de recueil (19) du filtrat, ladite poche étant reliée, par l'intermédiaire d'une tubulure et au niveau d'un orifice d'entrée, à un orifice de sortie (4) de l'unité de filtration (1).

10. Système à poches selon la revendication 9, **caractérisé en ce qu'**il comprend en outre une deuxième poche de recueil (34) qui est reliée à un orifice de sortie de la poche de collecte (28) par l'intermédiaire d'un filtre à déleucocyter (37) au moins un composant du fluide.

## Claims

1. Filtration unit (1) intended to allow the selective deleukocytation of a fluid containing blood platelets such as blood or a blood component, comprising an outer envelope (2) equipped with at least one inlet (3) and at least one outlet (4), the envelope containing a porous element (5) comprising a deleukocytation medium (8) by adsorption and/or filtration of leukocytes, **characterised in that** said medium consists of at least one non-woven layer (9) made of polyurethane that has been treated by gaseous plasma.

2. Filtration unit according to claim 1, **characterised in that** the average diameter of the pores of the non-woven layer(s) (9) ranges from 5 to 15 µm.

3. Filtration unit according to claim 1 or 2, **characterised in that** the deleukocytation medium (8) comprises several piled layers (9) that present a decreasing porosity in a continuous or slight manner in the direction of the flow (D) of the fluid.

4. Filtration unit according to any of claims 1 to 3, **characterised in that** the deleukocytation medium (8) also comprises at least one membrane placed downstream from said medium.

5. Filtration unit according to claim 4, **characterised in that** the membrane(s) is (are) formed from a material chosen from among the group comprising polymers of fluorocarbonated polymers, cellulose derivatives, polyurethanes, polysulphones, sulphonated polyethers, polycarbonate.

6. Filtration unit according to claim 5, **characterised in that** the membrane (s) is (are) formed from a polyvinylpyrrolidone/polysulphone co-polymer.

7. Filtration unit according to any of claims 4 to 6, **characterised in that** the pore diameter of the membrane(s) ranges from 2 to 10 µm.

8. Filtration unit according to any of claims 1 to 7, **characterised in that** the porous element (5) also comprises a pre-filter (10) and/or post-filter (18) that are made of at least one non-woven layer and that are placed on the downstream face and upstream face respectively of the deleukocytation medium (8).

9. System of pouches for the selective deleukocytation of a fluid containing blood platelets, **characterised in that** it comprises:
- a collecting pouch (28, 22) intended to receive the fluid for filtration, said pouch being connected, by means of tubing and at the level of an outlet, to an inlet (3) from a filtration unit (1) according to any of claims 1 to 8; and
- a filtrate recovering pouch (19), said pouch being connected, by means of tubing and at the level of an inlet, to an outlet (4) from the filtration unit (1).

10. System of pouches according to claim 9, **characterised in that** it also comprises a second collecting pouch (34) that is connected to an outlet from the collecting pouch (28) by means of a filter (37) to remove the leukocytes from at least one component of the fluid.

## Patentansprüche

1. Filterungseinheit (1), die dazu bestimmt ist, das selektive Entfernen von Leukozyten aus einer Blutplättchen enthaltenden Flüssigkeit, wie z. B. Blut oder einem Blutbestandteil, zu erlauben, mit einer mit wenigstens einer Eingangsöffnung (3) und wenigstens einer Ausgangsöffnung (4) versehenen äußeren Hülle, wobei die äußere Hülle (2) ein poröses Element (5) mit einem Milieu zum Entfernen von Leukozyten (8) durch Adsorption und / oder durch Ausfilterung der Leukozyten umschließt, **dadurch gekennzeichnet, dass** das genannte Milieu aus wenigstens einer nicht gewebten Schicht (9) auf Basis von Polyurethan gebildet wird, die durch gashaltiges Plasma behandelt wurde.

2. Filterungseinheit gemäß Anspruch 1, **dadurch gekennzeichnet, dass** der durchschnittliche Durchmesser der Poren der nicht gewebten Schicht(n) (9) zwischen 5 und 15 □m inbegriffen ist.

3. Filterungseinheit gemäß Anspruch 1 oder 2, **dadurch gekennzeichnet, dass** das Milieu zum Entfernen von Leukozyten (8) mehrere übereinander gestapelte Schichten (9) umfasst, die kontinuierlich oder nicht kontinuierlich in der Fließrichtung (D) der Flüssigkeit eine abnehmende Porosität aufweisen.

4. Filterungseinheit gemäß Anspruch 1 bis 3, **dadurch gekennzeichnet, dass** das Milieu zum Entfernen von Leukozyten (8) darüber hinaus wenigstens eine unterhalb des genannten Milieus platzierte Membran umfasst.

5. Filterungseinheit gemäß Anspruch 4, **dadurch gekennzeichnet, dass** die Membran(en) auf der Basis von einem aus der die Polymere von fluorkohlenstoffhaltigen Polymeren, Zellulosederivaten, Polyurethanen, Polysulfonen, sulfurierten Polyetheren, Polykarbonat umfassenden Gruppe ausgewählten Material gebildet wird / werden.

6. Filterungseinheit gemäß Anspruch 5, **dadurch gekennzeichnet, dass** die Membran(en) auf der Basis von einem Polyvinyl-/ Pyrolidon- / Polysulfon-Kopolymer gebildet wird / werden.

7. Filterungseinheit gemäß Anspruch 4 bis 6, **dadurch gekennzeichnet, dass** die Membran(en) einen zwischen 2 und 10 □m inbegriffenen Porendurchmesser aufweist / aufweisen.

8. Filterungseinheit gemäß Anspruch 1 bis 7, **dadurch gekennzeichnet, dass** das poröse Element (5) darüber hinaus einen Vorfilter (10) und / oder einen Nachfilter (18) aufweist, die in Form von wenigstens einer nicht gewebten Schicht gebildet werden und die jeweils unterhalb und oberhalb des Milieus zum Entfernen von Leukozyten (8) angeordnet sind.

9. Beutelsystem für das selektive Entfernen von Leukozyten aus einer Bluttplättchen enthaltenden Flüssigkeit, **dadurch gekennzeichnet, dass** es Folgendes umfasst:
- einen zur Aufnahme der zu filternden Flüssigkeit bestimmten Sammelbeutel (28, 22), wobei der genannte Beutel über einen Stutzen und auf der Höhe einer Ausgangsöffnung mit einer Eingangsöffnung (3) einer Filterungseinheit (1) gemäß Anspruch 1 bis 8 verbunden ist; und
- einen Auffangbeutel (19) des Filtrats, wobei der genannte Beutel über einen Stutzen und auf der Höhe einer Eingangsöffnung mit einer Ausgangsöffnung (4) der Filterungseinheit (1) verbunden ist.

10. Beutelsystem gemäß Anspruch 9, **dadurch gekennzeichnet, dass** es darüber hinaus einen zweiten Auffangbeutel (34) für wenigstens einen Bestandteil der Flüssigkeit umfasst, der mit einer Ausgangsöffnung des Sammelbeutels (28) über einen Filter zum Entfernen von Leukozyten (37) verbunden ist.
